# EUROPEAN PATENT APPLICATION

(11) **EP 4 647 713 A1**
(43) Date of publication of application: **12.11.2025**
(21) Application number: 24738410.0
(22) Date of filing: 04.01.2024
(51) Int. Cl.: F42B 27/00, F42B 12/36, F42B 8/26

(54) **TWO-STAGE NON-LETHAL ARTICLE MADE FROM A NATURAL CELLULOSE POLYMER, USES THEREOF AND METHOD FOR PRODUCING SAME, AND KIT**

(30) Priority: 05.01.2023 BR 102023000249
(71) Applicant: Condor S.A. Industria Quimica, 26053-640 - Nova Iguçu - RJ (BR)
(72) Inventor: QUEIROZ DE AGUIAR, Carlos Frederico, 26053-640 Nova Iguaçú - RJ (BR)
(74) Representative: Hoffmann Eitle
(86) International application number: PCT/BR2024/050002
(87) International publication number: WO 2024/145704

(57) **Abstract**

The present invention refers to a non-lethal article, which presents light and sound effects, with the body made of natural polymer, which provides during detonation the reduction of the quantity, size, and mass of the fragments, when compared to articles currently produced in flexible polymers, from petrochemical derivatives, thus mitigating the impacts arising from the projection, in addition to the generation of biodegradable cellulose fiber fragments. The present invention also refers to a method for the production of the non-lethal article, as well as its use, the use of a natural polymer, preferably cellulose fiber for the manufacture of a non-lethal article, as well as a kit comprising the non-lethal article. Preferably, the non-lethal item is a grenade.

## Description

### TECHNICAL FIELD

This invention refers to a non-lethal article that presents light and sound effects, with the body made of natural polymer, which provides, during its operation, the reduction of the quantity, size, and mass of the fragments, when compared to articles currently produced from flexible polymers, derived from petrochemical derivatives, thus mitigating the impacts arising from the projection, in addition to the generation of biodegradable cellulose fiber fragments.

The invention also refers to the method of production of the aforementioned article.

More precisely, the invention refers to a non-lethal grenade, which features light and sound effects, with a body made of cellulose fiber.

### STATE OF THE ART

"Non-lethal technologies" also known as "less lethal technologies" are designed and produced for temporary personal and material incapacitation, without causing deaths, permanent injuries or even undesirable material damage to buildings and the environment. It gives the operators thereof the ability to reduce occasions where the use of a lethal weapon is required. Thus, non-lethal technologies are means to ensure law enforcement, which allow the use of the gradual and proportional use of force, limiting and reducing the use of means that can cause death or serious injury.

Non-lethal technologies also have wide application in the area of public security, especially in the control of disturbances of all kinds, including in the case of rebellions in the prison system.

The Applicant is a national company, considered a "Strategic Defense Company (SDC)", which for more than 35 years has been operating in the production of equipment, non-lethal technologies for riot control and pyrotechnics for use in signaling and salvage, whose objective is the preservation of lives and is among the leading export companies in the defense sector in Brazil.

In recent decades, society and its relations have changed and new conflicts have emerged, requiring a new behavior from security agents around the world. The United Nations (UN) recommends the need for restrictions on the use of force and firearms, and governments are advised to employ non-lethal weapons and technologies to their security forces. A 1990 UN resolution recommends, to the entities responsible for law enforcement, the use of non-lethal weapons with the aim of reducing the use of means that could cause death or injury. However, in that same resolution, the UN recommends that the use of these weapons should be controlled in order to minimize possible risks. Thus, even in the case of a non-lethal technology, safety in handling must be taken into account, so that it is used only by trained people and that it is not used incorrectly.

Within this scenario, the Applicant adopts the concept of progressive, proportional and selective use of force, such as, for example, technologies that generate explosions and secondary effects such as luminosity and effect of incapacitating agents, etc. One of these technologies employed are the non-lethal explosive grenades of light and sound for use *outdoor* and *indoor,* with bodies produced in flexible polymer, which produce shrapnel in flexible material.

PI 1002280-5 B1, granted to the Applicant itself, discloses a hand grenade that allows the location of the artifact or fragments associated with it after it has been activated and a method of manufacturing a hand grenade trigger, according to which, a housing for an automatic identification device is provided in the body of the trigger and then an equally cylindrical automatic identification device is inserted inside said housing. However, the grenade disclosed in PI 1002280-5 B1 has a body made of hardened polymer and does not mention or even suggest the possibility of using another material for manufacturing its body.

KR101950760B1 discloses a biodegradable grenade for training, whose body is made of an *eco-friendly* material produced from natural minerals. Ecological minerals, including natural minerals, martensite, silica sand, and loess that can be spontaneously decomposed, are mixed with a binder and water, stirred, and subjected to a primary calcination treatment through a mold of a training grenade body to form a grenade body. However, KR101950760B1 does not mention or even suggest the possibility of using a cellulose material to manufacture the body of said biodegradable grenade.

KR100427221B1 discloses an eco-friendly hand grenade for training and a method of making it to improve the efficiency of the explosion, prevent injury to a user, and train a large number of people for a short period of time by making the hand grenade from naturally decomposed mineral. In particular, the body of the KR100427221B1 grenade is made of a mixture of mineral compounds including as raw material 80 to 90% by weight of barite, 5 to 10% by weight of tungsten, 0.1 to 0.5% by weight of silica and 2 to 3% by weight of loess. However, KR100427221B1, as well as KR101950760B1, does not mention or even suggest the possibility of using a cellulose material for the manufacture of the body of the aforementioned biodegradable grenade.

Therefore, the non-lethal explosive grenades currently produced with bodies made of flexible polymer ("from petrochemical derivatives") and composite fibers, when detonated project the EOT type trigger ("fuze") (time warhead fuze) during the occurrence of the detonation, making this component a true projectile, which when hitting the offender can cause serious and/or irreversible injuries. In addition, they can produce large fragments with a mass of more than 5 grams at a distance of more than 5 meters, which is currently a limiting control specification parameter for this type of product in the national territory. Another aspect is that these current models of grenades do not make it possible to track them after detonation. In this way, the objective of this invention is to provide a non-lethal grenade, which presents light and sound effects, with a body made of cellulose fiber and that can be tracked, in order to increase operational safety and mitigate environmental impacts, providing greater sustainability of the product in line with the guidelines of the 2030 agenda.

### SUMMARY OF THE INVENTION

The present invention refers to a non-lethal grenade, which presents light and sound effects, with a body made of cellulose fiber (natural polymer), which provides, during detonation, the reduction of the quantity, size, and mass of the fragments, when compared to the grenades currently produced in flexible polymers, from petrochemical derivatives, mitigating the impacts arising from the projection, in addition to the generation of biodegradable cellulose fiber fragments. Added to this is the fact of the use of a delay mix specially developed for the EOT (time warhead fuze) type trigger, which does not have "lead compounds" in its composition. In addition, the EOT (time warhead fuze) trigger, which can optionally contain an I-REF chip, enables traceability even after use.

The non-lethal explosive grenades currently known in the market and produced with bodies made of flexible polymer ("from petrochemical derivatives") and cellulose fiber, when operating project the EOT (time warhead fuze) type trigger during the occurrence of the detonation, making this component a true projectile, which when hitting the offender can cause serious irreversible injuries. In addition, they can produce large fragments with a mass of more than 5 grams at a distance of more than 5 meters, which is currently a limiting control specification parameter for this type of product in the national territory. Another aspect is that these current models do not make it possible to track them after detonation.

The grenade of this invention, with a dual-stage (GL-307/ECO) and with a body and load holder made of "natural polymer" (cellulose fiber), is equipped with an EOT (time warhead fuze) trigger with a dual-stage system, which ejects the trigger before detonation of the body (operation called "depoting"), with the objective of preventing this component from becoming a projectile and allowing its traceability. The grenade provides an efficient action in the theater of operations, with the generation of small fragments with a mass equal to or less than 5 grams. Optionally, the EOT (time warhead fuze) contains a small tamper-proof I-REF chip, which works by radio frequency, allowing traceability of the grenade even after it has been operational. In addition, this EOT option with chip is essential to combat cargo theft and diversion of materials, situations in which stolen weapons from the arsenals of security forces are used by criminals and become, with the implantation of this chip, more easily detected.

The body and load holder of the grenade, both made of natural polymer ("cellulose fiber") contain, respectively, flash ("flash mass") and explosive ("shot mass") chemical masses and an EOT-type trigger ("fuze") with delayed mass ("mixed delay") compacted without lead compounds in their composition. Both are connected by means of a mechanical component in flexible polymeric material, called the EOT housing, which has a delay holder in rigid polymeric material and loose black powder propellant inside. The EOT housing allows a perfect union between the EOT, the grenade body and the explosive load holder, providing sealing and the ejection of the EOT ("depoting" operation) before detonation of the body.

In order to satisfactorily achieve the objectives of this invention, a grenade is provided with:
- EOT-type trigger ("fuze") that is responsible for the initial firing of the grenade and the delay of the first stage;
- Body, which is the mechanical component with a natural polymer structure ("cellulose fiber"), which contains a chemical mass of flash ("flash mass"), responsible for the emission of luminosity;
- Load holder, which is the mechanical component with a natural polymer structure ("cellulose fiber") that contains the explosive chemical mass ("shot mass"), responsible for the emission of sound ("sound pressure");
- EOT housing, which is a mechanical component with a polymeric structure in flexible material, which allows a perfect union between the EOT, the body of the grenade and the load holder, providing the sealing and ejection of the EOT ("depoting" operation) before detonation of the body;
- Delay port of the EOT housing made of rigid polymeric material that is responsible for the delay of the second stage.

### BRIEF DESCRIPTION OF THE DRAWINGS

The present invention will now be described in more detail, but only by way of example, with reference to the accompanying drawings, in which:

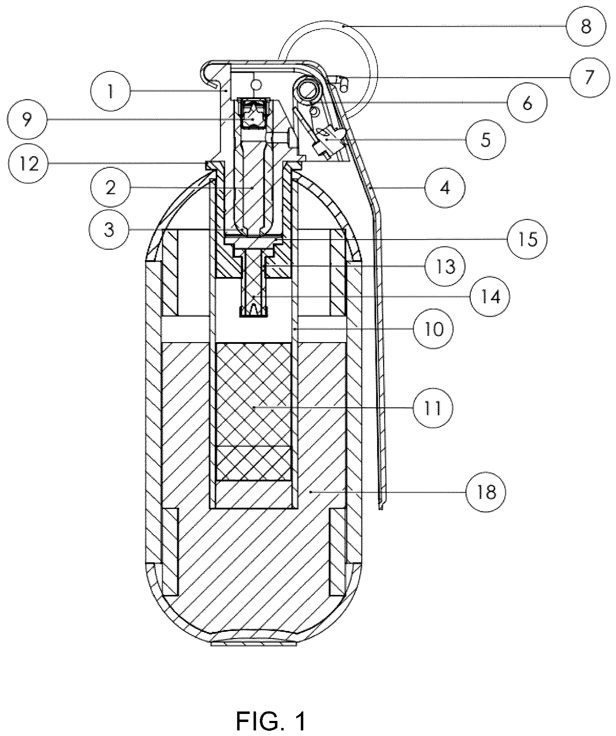

**Figure 1** shows a sectional view of the Non-Lethal Explosive Grenade in natural polymer (cellulose fiber), which is composed of an EOT (Time Warhead Fuze) type actuator in polymer body 1. This actuator has a chemical delay mix without lead compounds in its composition 2, compacted inside a metal insert called a delay gate 3, a metal handle 4, a metal striker 5, a torsion spring 6, a metal safety clamp 7, a metal ring 8 and a mechanical percussion fuze 9.

The EOT (Time Warhead Fuze) type actuator is joined to a load holder 10, made of natural polymer ("cellulose fiber") that contains a low-velocity explosive mixture 11 called shot mass ("white powder"), responsible for generating sound ("sound pressure") during its burning ("combustion reaction") by means of a "mechanical component" composed of "flexible polymeric material" called housing of the EOT 12. Inside the EOT housing, there is a retardation holder made of rigid polymeric material 13, which has a low-velocity explosive, "black powder", pressed 14. Inside the housing, there is also a quantity of loose black powder 15.

The subassembly formed by the EOT (Time Warhead Fuze) type trigger, the explosive load holder and the cover is bonded by means of adhesive to the subassembly of the grenade body containing flash chemical mass.

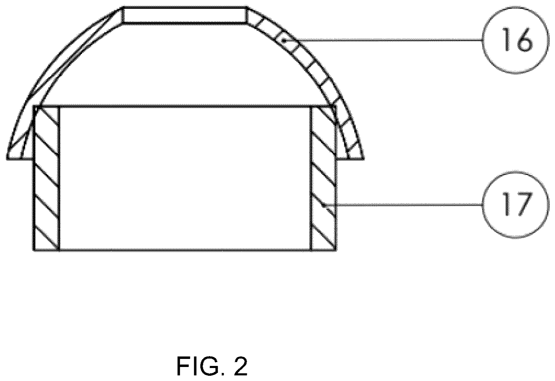

The subassembly formed by the EOT, EOT housing and the load holder is fixed by means of adhesive to the subassembly of the grenade cover **(****Figure 2****),** which is formed by the union of a mechanical component 16 with semi-spherical geometry (top cover) and another component 17 with cylindrical geometry (belt) by means of adhesive, both components made of natural polymer ("cellulose fiber") waterproofed by means of specific sealant

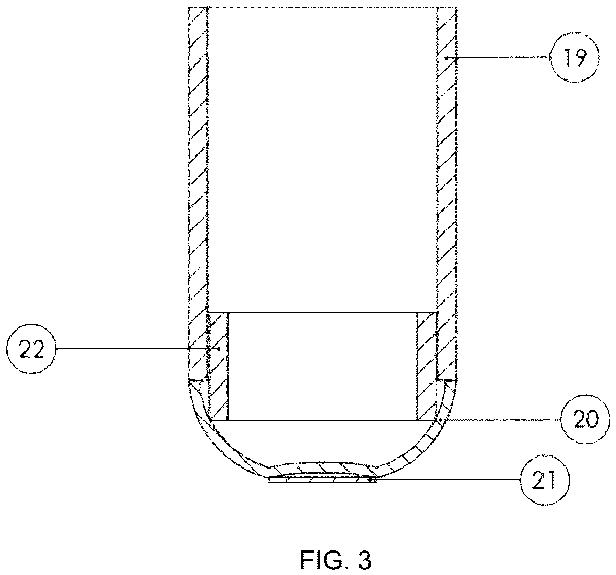

This new subassembly is attached by means of adhesive to the subassembly of the grenade body **(****Figure 3****),** which, as already described in **Figure 1****,** contains a flash mass 18 inside.

**Figure 3** shows the subassembly of the grenade body, which is formed by the union of mechanical component 19 with cylindrical geometry (side strap) to the subassembly of the lower cover, which is formed by the union of a mechanical component 20 with semi-spherical geometry (lower cover) and a disc 21 at the base and another component 22 with cylindrical geometry (strap). All components are made of natural polymer ("cellulose fiber") waterproofed by means of a specific sealant and fixed by means of adhesive.

After the assembly stage, the labeling is carried out, using a sleeve type shrink label and then the previously assembled EOT (time warhead fuze) actuator (fuze) is inserted with sealant in the flexible housing positioned on the top cover of the grenade. Inside the housing there is a quantity of loose black powder 15, which when burned ("combustion reaction") generates gases and consequent increase in internal pressure, responsible for the ejection (operation called "depotage") of the EOT (1) and the ignition of the retardant holder 13 made of rigid polymeric material ("second stage retardant"), which contains pressed black powder 14 inside, responsible for the ignition of explosive mass 11, which generates sound ("sound pressure"), which is inside the load holder 10 and subsequent ignition of flash mass 18, "light intensity generator", which is inside the body of the grenade.

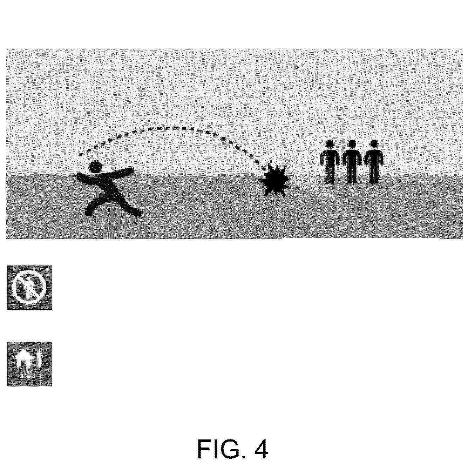

**Figure 4** refers to a demonstration of the use of this invention. The non-lethal explosive grenade must be thrown in the direction of law-breakers at a minimum safety distance of 10 meters.

With the handle of the grenade firmly held in the palm of one hand, the safety pin is removed with the other hand and then the grenade is thrown in the direction of the offender(s) at a minimum safety distance of 10 meters. During the trajectory, the handle is then released by the action of the firing pin, which moves to perform the percussion of the mechanical fuze. The fire of the fuse ignites the column of chemical retardant mass that is pressed in the metal retardation holder and after its end of burning, ignites the propellant black powder ("depoting powder") that is loose inside the EOT housing, which when burning generates gases and due to the consequent increase in internal pressure ejects the EOT (operation called "depotage") from its cavity and ignites the second delay of black powder pressed in the plastic retardation holder, which after its end ("burning"), finally promotes the ignition of the explosive mass, which generates sound ("sound pressure") and immediately ignites the flash mass, which generates luminosity.

### DETAILED DESCRIPTION OF THE EMBODIMENTS OF THE INVENTION

The non-lethal explosive grenades currently produced on the market feature:
- Flexible polymer body (derived from petrochemical derivatives), which, when functioning, project fragments with a mass greater than 5 grams at a distance greater than 5 meters, which is a limiting control specification parameter for this type of product in the national territory, allowing the occurrence of injury. In addition, **some models project** the EOT (time warhead fuze) type trigger ("fuze") during the occurrence of the detonation, making it a true projectile, which when hitting the offender can cause serious irreversible injuries. What is more is the fact that these models do not allow tracking thereof after detonation;
- They project fragments with a mass of more than 5 grams at a distance of more than 5 meters;
- They project the EOT (time warhead fuze) type trigger during the occurrence of the detonation, making it a true projectile, which when hitting the offender can cause serious irreversible injuries;
- The current models known in the market do not allow it to be traced after detonation.

The present invention therefore aims to solve these technical problems/disadvantages of the products currently on the market.

The grenade of this invention, designated GL-307/ECO, with a body and load holder made of "natural polymer" ("cellulose fiber"), is equipped with an EOT (time warhead fuze) actuator and a dual-stage system, which ejects the EOT ("fuze") type actuator (time warhead fuze) before the detonation of the body, in order to prevent this component from becoming a projectile. The grenade provides an efficient action in the theater of operations, with the generation of small fragments with a mass equal to or less than 5 grams. Optionally, it contains a small tamper-proof I-REF chip, which works by radio frequency, allowing traceability of the grenade even after its operation. In addition, this innovation is essential to combat cargo theft and diversion of materials, situations in which weapons stolen from the police are used by criminals and become, with the implantation of this chip, more easily clarified.

There are non-lethal explosive grenades produced in a metallic body, which have a flexible polymer or natural polymer load holder, containing explosive chemical mass. These grenades are used in tactical operations to enter premises ("indoor grenades") and occasionally, some suppliers or customers use them in outdoor disturbance control operations. They have the same principle of manual activation and when activated, they produce sound ("sound pressure") and luminosity intensity. However, the luminosity produced is lower than the luminosity of the proposed development, object of this invention, because the energy source comes only from the burning (combustion reaction) of the chemical mass contained inside the load holder. Furthermore, after their operation, the bodies can be thrown back by the aggressors against the operators, or against the demonstrators involved in the theater of operations, which can produce serious irreversible injuries, including death, in addition to material damage, when thrown against existing properties during urban conflicts.

A preferred modality provides a grenade equipped with:
- EOT-type trigger ("fuze") is responsible for the initial firing of the grenade and the delay of the first stage;
- Body, which is the mechanical component with a natural polymer structure ("cellulose fiber"), which contains a load of innocuous powder (industrial talc) (GL-304/ECO);
- Load holder, which is the mechanical component with a natural polymer structure ("cellulose fiber") that contains the explosive chemical mass ("shot mass"), responsible for the emission of sound ("sound pressure");
- EOT housing, which is a mechanical component with a polymeric structure in flexible material, which allows a perfect union between the EOT, the body of the grenade and the load holder, providing the sealing and ejection of the EOT ("depoting" operation) before detonation of the body;
- Retardant holder of the EOT housing made of rigid polymer material is responsible for the delay of the second stage.

A second preferred embodiment provides a grenade equipped with:
- EOT-type trigger ("fuze") is responsible for the initial firing of the grenade and the delay of the first stage;
- Body, which is the mechanical component with a natural polymer structure ("cellulose fiber"), which contains a disabling agent (preferably Orthochlorobenzalmalononitrile - CS) (GL-305/ECO);
- Load holder, which is the mechanical component with a natural polymer structure ("cellulose fiber") that contains the explosive chemical mass ("shot mass"), responsible for the emission of sound ("sound pressure");
- EOT housing, which is a mechanical component with a polymeric structure in flexible material, which allows a perfect union between the EOT, the body of the grenade and the load holder, providing the sealing and ejection of the EOT ("depoting" operation) before detonation of the body;
- Retardant holder of the EOT housing made of rigid polymer material is responsible for the delay of the second stage.

A third preferred modality provides a grenade equipped with:
- EOT-type trigger ("fuze") is responsible for the initial firing of the grenade and the delay of the first stage;
- Body, which is the mechanical component with a natural polymer structure ("cellulose fiber"), which contains a load of incapacitating agent (natural capsaicin and/or PAVA) (GL-308/ECO);
- Load holder, which is the mechanical component with a natural polymer structure ("cellulose fiber") that contains the explosive chemical mass ("shot mass"), responsible for the emission of sound ("sound pressure");
- EOT housing, which is a mechanical component with a polymeric structure in flexible material, which allows a perfect union between the EOT, the body of the grenade and the load holder, providing the sealing and ejection of the EOT ("depoting" operation) before detonation of the body;
- Retardant holder of the EOT housing made of rigid polymer material is responsible for the delay of the second stage.

The present invention also refers to the method of producing of the mechanical components of the grenade body made of natural polymer ("cellulose fiber"), which are produced using cylindrical tools such as mandrel (straps), stamping (disc), hot pressing process, thermocompression (cover and base) and cutting and gluing process. The mechanical components of the natural polymer grenade body ("cellulose fiber") can also be produced by means of a cellulose pulp molding process, followed by a gluing operation.

### Process of Cutting and Gluing the Mechanical Components of the Grenade

The components made of cellulose fiber (natural polymer) are obtained by different manufacturing processes:
Covers: thermocompression process.
Straps (side and internal): process of winding the raw material into a dedicated cylindrical tool ("mandrel"), followed later by cutting with the objective of producing mechanical components with a cylindrical shape in the specified dimensions (diameters and length).

### Obtaining the Domes (top and bottom cover)

Domes (mechanical components of the grenade with semi-spherical geometry) are obtained by overlaying small strips of paper (for example: kraft) saturated with viscous adhesive, arranged radially forming a "star" (when viewed from above) that is compressed into a rigid matrix with a spherical shape, acquiring this new geometry through a thermocompression process.

### Obtaining the Straps (side and internal):

Belts with cylindrical geometry are obtained by radial winding and overlapping of paper layers (for example: Kraft) with the addition of viscous adhesive. This process is carried out by means of specific machinery that collects the cellulose sheets (which have different thicknesses) and unifies them forming a cylinder of great length. This "resulting cylinder" is cut by a cutting tool that acts on the length of the latter, converting it into smaller cylinders (already with the desired working dimension).

### Ending

The inner straps are glued to the domes with industrial adhesive. For the top covers, drilling is carried out by means of a cutting die attached to a hand tool. On the lower covers, instead of cutting in its "dome", there is a flattening that is performed by manual pressing tool with subsequent disc gluing to reinforce this surface. Finally, the lower covers are glued to the bodies (external straps) with industrial adhesive configuring a subassembly called "body" of the grenade".

### Molded Cellulose Pulp Process

In this process, unlike the cutting and gluing process in which the mechanical components are obtained by means of a cylindrical tool (mandrel type), "thermocompression" and stamping, the raw material used is clean cellulose fiber shavings. **In** a first step, these shavings are crushed in a cylinder with the objective of promoting the breakdown of cellulose fibers. Then, the crushed material goes to a tank in order to promote the obtaining of a diluted pulp. The more dilute the pulp, the better the finish of the mechanical component obtained.

Subsequently, the cellulose pulp is sucked into a mold, thus obtaining the mechanical component with the customized geometry. The molds are usually made of aluminum and coated with a stainless steel screen. **In** this stage, the weight of the mechanical component is worked. The denser the pulp solution, the greater the strength of the mechanical component.

Then follows the drying stage, which can be by gas oven, greenhouse or mechanical ventilation.

The last stage is forming, where the part is finished in a press, with the aim of promoting the final shape. In this process, it is possible to place reliefs to identify the piece, such as logos and codes.

This invention also refers to the use of the non-lethal article in operations in disturbance control operations in *outdoor* environments and, eventually, tactical operations of entering premises (*"indoor* grenades").

The present invention also refers to the use of a natural polymer, preferably cellulose fiber, for the manufacture of a non-lethal article, preferably in which the non-lethal article is a grenade.

The present invention also refers to a kit, which comprises: (a) the non-lethal article of the present invention, and (b) instructions for use by the user.

It should be noted that the embodiments and features in the context of one of the aspects of the present invention also apply to other aspects of the invention. Although embodiments have been disclosed in the specification with reference to specific examples, it will be recognized that the invention is not limited to those modalities. Various modifications may become evident to those skilled in the art and may be acquired from the practice of the invention, and such variations are considered within the broad scope of the present invention. It should be understood that the materials used and the chemical details may be slightly different or modified from the descriptions without departing from the methods and compositions disclosed and taught by the present invention.

Additional aspects of the invention and preferred characteristics thereof are provided in the claims of the present invention.

### TESTS

### Assay for the evaluation of grenade fragmentation

The test was carried out with the participation of the Quality Assurance sector, with the objective of evaluating the mass of the fragments from the GL-307/ECO grenade body during its detonation.

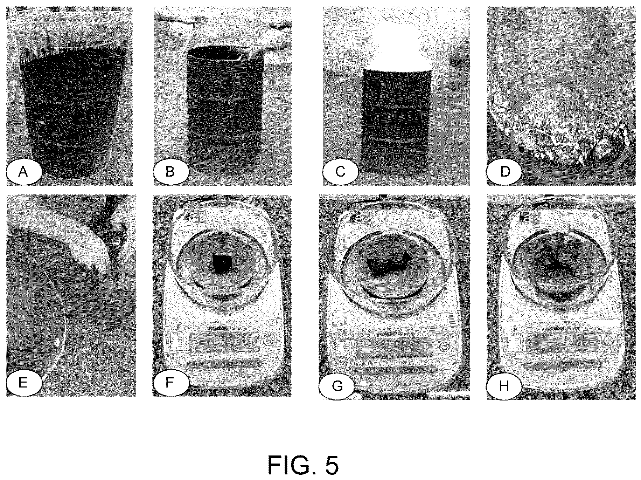

Inside a restrictor element (50 L carbon steel barrel), 3 units of GL 307/ECO were activated (one at a time) with the objective of analyzing the fragments generated after the detonation of the product. These fragments were gathered into 3 packages and then weighed on a precision scale **(****Figure 5****).**

### Result

The fragment with the highest mass found was 4.58 g referring to the housing of the EOT/EOT bushing (¹), which is the mechanical component responsible for the union of the EOT with the grenade body, as well as for the depotation of the EOT during detonation. The other elements generated from the body reached a maximum value of 3.63 g and a minimum of 1.78 g. Thus, the grenade present invention, when triggered, fully complies with the provisions of the current technical standards regarding the project's input specification (≤ 5.0 g).
(1) - Trigger housing ("EOT bushing")
   - Material: black PVC compound D48/1
   - Hardness: 50 Shore A.

### Conclusion

The GL-307/ECO explosive grenade proved to be a promising product, meeting the requirements/conditions of the design boundary: low mass for the fragments found (≤ 5.0 g), according to tests carried out during its development, breaking the plant's dependence on petroleum-derived polymers. Combining high performance in the field (100% in performance tests based on the premises of the Brazilian Army Technical Standard NEB/T E-321A, recommended in Ordinance N°. 189-EME, August 18, 2020 and Ordinance N°. 118 - COLOG, October 4, 2019) and low tooling cost associated when directly compared to the traditional plastic injection process.

## Claims

1. A non-lethal article **characterized by** comprising:
- main body and load holder made of natural cellulose fiber polymer,
- an EOT (time warhead fuze) type trigger, and
- dual-stage system.

2. The non-lethal article according to claim 1, **characterized in that** it is a grenade.

3. The non-lethal article according to claim 1 or 2, **characterized in that** the load to be released consists of sound and light.

4. The non-lethal article according to claim 1 or 2, **characterized in that** the cargo to be released consists of innocuous powder, preferably industrial talc.

5. The non-lethal article according to claim 1 or 2, **characterized in that** the load to be released consists of a disabling agent, optionally in which the disabling agent is orthochlorobenzalmalononitrile (CS), or natural capsaicin and/or PAVA.

6. The non-lethal article according to any of claims 1 to 5, **characterized in that** it further comprises a tamper-proof I-REF chip.

7. The non-lethal article according to any of claims 1 to 5, **characterized in that** when the grenade is detonated, it generates a reduction in the quantity, size and mass of the fragments.

8. A method for producing mechanical components of the non-lethal article body made of natural cellulose fiber polymer, as defined in any of claims 1 to 6, **characterized in that** it comprises the use of cylindrical mandrel tools (strapping), stamping (disc), followed by a hot pressing process, thermocompression (lid and base) and a cutting and gluing process, optionally the mechanical components of the grenade body made of natural cellulose fiber polymer are produced through the process of molding the cellulose pulp, followed by a gluing operation.

9. Use of a non-lethal article, as defined in any of claims 1 to 6, **characterized in that** it is in the area of public security, especially in the control of disturbances of all kinds.

10. Use of a natural polymer, preferably cellulose fiber, **characterized in that** it is for the manufacture of a non-lethal article, as defined in any of claims 1 to 6, to mitigate the impacts arising from the projection, and to generate biodegradable cellulose fiber fragments.

11. Kit, **characterized by** comprising:
(a) the non-lethal article, as defined in any of claims 1 to 6, and
(b) instructions for use by the user.

## Amended claims

### Amended claims under Art. 19.1 PCT

1. A non-lethal article **characterized by** comprising:
- body of the grenade (19, (20), (21), (22) and load holder (10) made of natural cellulose fiber polymer,
- an EOT (time warhead fuze) type trigger (1), and
- dual-stage system.

2. The non-lethal article according to claim 1, **characterized in that** it is a grenade.

3. The non-lethal article according to claim 1 or 2, **characterized by** the fact that the load to be released consists of sound and light.

4. The non-lethal article according to claim 1 or 2, **characterized by** the fact that the cargo to be released consists of innocuous powder, preferably industrial talc.

5. The non-lethal article according to claim 1 or 2, **characterized by** the fact that the load to be released consists of a disabling agent, optionally in which the disabling agent is orthochlorobenzalmalononitrile (CS), or natural capsaicin and/or PAVA.

6. The non-lethal article according to any of claims 1 to 5, **characterized by** the fact that it further comprises a tamper-proof I-REF chip.

7. The non-lethal article according to any of claims 1 to 5, **characterized by** the fact that when the grenade is detonated, it generates a reduction in the quantity, size and mass of the fragments.

8. A method for producing mechanical components of the non-lethal article body made of natural cellulose fiber polymer, as defined in any of claims 1 to 6, **characterized by** the fact that it comprises the use of cylindrical mandrel tools (strapping), stamping (disc), followed by a hot pressing process, thermocompression (lid and base) and a cutting and gluing process, optionally the mechanical components of the grenade body made of natural cellulose fiber polymer are produced through the process of molding the cellulose pulp, followed by a gluing operation.

9. Use of a non-lethal article, as defined in any of claims 1 to 6, **characterized by** the fact that it is in the area of public security, especially in the control of disturbances of all kinds.

10. Use of a natural polymer, preferably cellulose fiber, **characterized by** the fact that it is for the manufacture of a non-lethal article, as defined in any of claims 1 to 6.

11. Kit, **characterized by** comprising:
(a) the non-lethal article, as defined in any of claims 1 to 6, and
(b) instructions for use by the user.
